# EUROPEAN PATENT APPLICATION

(11) **EP 3 260 550 A1**
(43) Date of publication of application: **27.12.2017**
(21) Application number: 17179514.9
(22) Date of filing: 12.03.2009
(51) Int. Cl.: C12Q 1/02, C12M 1/00, G01B 11/00, G01N 21/17, G06T 7/246, G06T 7/12, G06K 9/00, C12M 1/36, C12M 1/34

(54) **METHOD FOR ANALYZING IMAGE FOR CELL OBSERVATION, IMAGE PROCESSING PROGRAM, AND IMAGE PROCESSING DEVICE**

(30) Priority: 24.03.2008 JP 2008075669
(62) Divisional of application: 09724786.0
(71) Applicant: Nikon Corporation, Minato-ku, Tokyo 108-6290 (JP)
(72) Inventor: Mimura, Masafumi, Tokyo, 108-6290 (JP); Ito, Kei, Tokyo, 108-6290 (JP)
(74) Representative: Hoffmann Eitle

(57) **Abstract**

The contour of a cell being observed is extracted from a cell observation image obtained by an imaging device, and a cell model made by modeling the outer shape of the cell is adapted. The direction in which the cell being observed is predicted to move is derived using the adapted cell model.

## Description

### TECHNICAL FIELD

Various embodiments described herein relate to image processing means in cell observation for deriving a predicted movement direction of cells.

### TECHNICAL BACKGROUND

Image processing techniques for predicting how a moving object will move in the future by processing and analyzing images of the photographed moving object are widely used as means for predicting the movement of a moving object. Linear prediction, a Kalman filter, and other chronological prediction based on past movement distance are used in such movement prediction (e.g., see Patent Document 1).
Patent Document 1: Japanese Laid-open Patent Publication No. 2007-303886

### DISCLOSURE OF THE INVENTION

### PROBLEMS TO BE SOLVED BY THE INVENTION

However, when the observation target is a cell, the movement of the cell is frequently rapid, static, or the like, and linear prediction is often difficult. A particle filter or the like that can make nonlinear predictions has been considered as a prediction method for cases in which linear prediction is difficult, but there is a problems in that the precision is as yet not considered to be sufficient for predicting movement that is mostly random.

Since conventional movement prediction is carried out by processing numerous time-lapse images to predict future movement directions, there is a problem in that a heavy processing load is placed on the image processing device, and the size and cost of the image processing device are increased in order to carry out rapid prediction processing.

The present invention was developed in view of the problems described above, and an object of the present invention is to provide means capable of predicting cell movement using a simple configuration.

### MEANS TO SOLVE THE PROBLEMS

In accordance with a first aspect exemplifying the present invention, there is provided a method for analyzing an image for cell observation, comprising: obtaining an observation image in which an observed cell has been photographed by an imaging device; extracting a contour of the observed cell from the obtained observation image; adapting a cell model made by modeling an outer shape of the cell to the observed cell from which the contour has been extracted; and deriving a movement direction in which the observed cell is predicted to move, using the adapted cell model.

In accordance with a second aspect exemplifying the present invention, there is provided a method for analyzing an image for cell observation, comprising: obtaining an observation image in which an observed cell has been photographed by an imaging device; extracting a contour of the observed cell from the obtained observation image; computing bias of the internal structure in relation to the contour shape of the observed cell from which the contour has been extracted; and deriving a movement direction that the observed cell is predicted to move based on the computed bias of the internal structure.

In accordance with a third aspect exemplifying the present invention, there is provided a program for processing an image for cell observation, comprising: obtaining an observation image in which an observed cell has been photographed by an imaging device; extracting a contour of the observed cell from the obtained observation image; adapting a cell model made by modeling an outer shape of the cell to the observed cell from which the contour has been extracted; deriving a movement direction in which the observed cell is predicted to move, using the adapted cell model; and outputting to the exterior the derived movement direction of the observed cell.

In accordance with a fourth aspect exemplifying the present invention, there is provided a program for processing an image for cell observation, comprising: obtaining an observation image in which an observed cell has been photographed by an imaging device; extracting a contour of the observed cell from the obtained observation image; computing bias of the internal structure in relation to the contour shape of the observed cell from which the contour has been extracted; deriving a movement direction in which the observed cell is predicted to move based on the computed bias of the internal structure; and outputting to the exterior the derived movement direction of the observed cell.

In accordance with a fifth aspect exemplifying the present invention, there is provided an image processing device for cell observation, comprising an imaging device configured to photograph an observed cell; an image analysis section obtaining an observation image in which the observed cell has been photographed by the imaging device, and deriving a movement direction in which the observed cell is predicted to move; and an output section for outputting to the exterior the movement direction of the observed cell derived by the image analysis section, wherein the image analysis section is configured to extract a contour of the observed cell from the observation image, to adapt a cell model made by modeling an outer shape of the cell to the observed cell from which the contour has been extracted, and to derive a movement direction in which the observed cell is predicted to move, using the adapted cell model.

In accordance with a sixth aspect exemplifying the present invention, there is provided an image processing device for cell observation, comprising an imaging device configured to photograph an observed cell; an image analysis section obtaining an observation image in which the observed cell has been photographed by the imaging device, and deriving a movement direction in which the observed cell is predicted to move; and an output section outputting to the exterior the movement direction of the observed cell derived by the image analysis section, wherein the image analysis section is configured to extract a contour of the observed cell from the observation image, compute a bias of the internal structure in relation to the contour shape of the observed cell from which the contour has been extracted, and derive a movement direction that the observed cell is predicted to move based on the computed bias of the internal structure.

### ADVANTAGEOUS EFFECTS OF THE INVENTION

In accordance with such a method for analyzing an image for cell observation, an image processing program, and an image processing device, the movement direction of a cell can be predicted from a single image photographed by an imaging device. Therefore, it is possible to provide means capable of predicting cell movement using a simple configuration.

### BRIEF DESCRIPTION OF THE DRAWINGS

FIG. 1 is a conceptual view of the movement prediction method for deriving a movement direction from the position of an observed cell that projects from a cell model;
FIG. 2 is a schematic view of a culture observation system showing an application example of the present invention;
FIG. 3 is a block diagram of the culture observation system;
FIG. 4 is a schematic diagram illustrating the state of contour extraction processing for extracting the contour of a cell;
FIG. 5 is a conceptual view of the movement prediction method for deriving a movement direction from the cell model adapted to an observed cell;
FIG. 6 is a conceptual view of the movement prediction method for deriving a movement direction from the offset between the center of gravity of the cell model and the center of gravity of the observed cell;
FIG. 7 is a conceptual view of the movement prediction method for deriving a movement direction from the density distribution of the observed cell;
FIG. 8 is a block view showing the general configuration of the image processing device;
FIG. 9 is a flowchart showing the main flow in the image processing program;
FIG. 10 is a flowchart that corresponds to the prediction algorithm A selected in the main flow;
FIG. 11 is a flowchart that corresponds to the prediction algorithm B selected in the main flow;
FIG. 12 is a flowchart that corresponds to the prediction algorithm C selected in the main flow; and
FIG. 13 is a configuration example of the display image of the cell movement tracking interface displayed on the display panel in the case that the image processing program is executed.

### EXPLANATION OF NUMERALS AND CHARACTERS

BS: Culture observation system
GP: Image processing program
C: Observed cell
54: Macro viewing system
54c: Imaging device
55: Microscope viewing system
55c: Imaging device
100: Image processing device
120: Image analysis section
130: Output section

### DESCRIPTION OF THE PREFERRED EMBODIMENTS

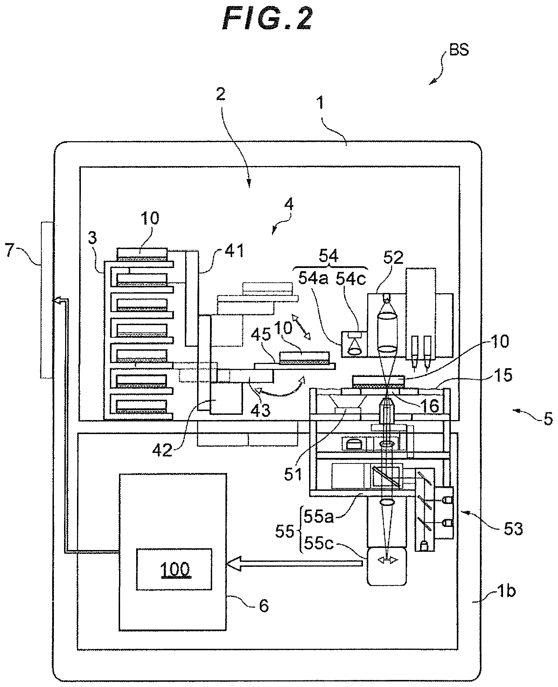

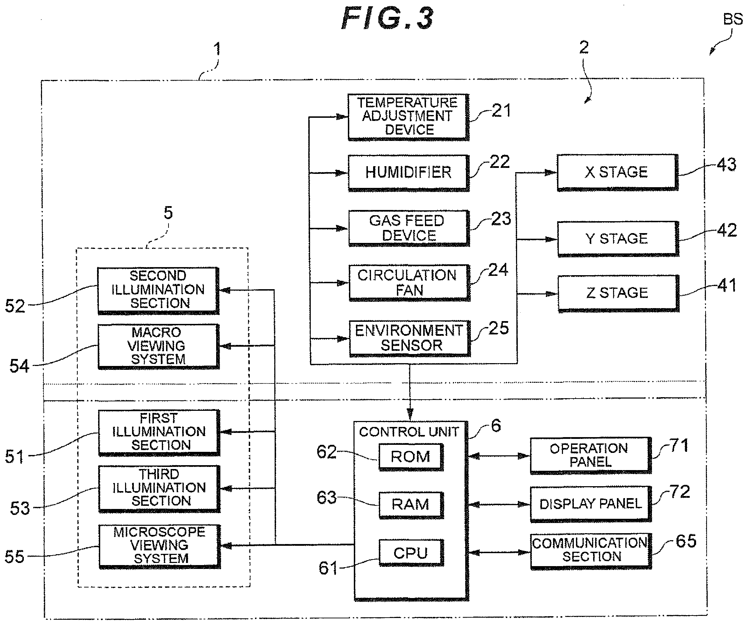

The embodiments of the present invention are described below with reference to the drawings. FIGS. 2 and 3 show a schematic diagram and a block diagram, respectively, of a culture observation system as an example of a system to which the image processing device for cell observation of the present invention has been applied.

In terms of overall structure, the culture observation system BS is composed of a culture chamber 2 disposed in the upper part of a casing 1, a shelved stocker 3 for storing and holding a plurality of culture containers 10, an observation unit 5 for observing samples inside the culture containers 10, a transport unit 4 for transporting the culture containers 10 between the stocker 3 and the observation unit 5, a control unit 6 for controlling the operation of the system, and a control board 7 provided with an image display device.

The culture chamber 2 is a chamber for forming and maintaining a culture environment corresponding to the species, purpose, and other attributes of the cells to be cultured; and the chamber is kept in an airtight state after samples have been loaded in order to prevent changes in the environment and contamination. The culture chamber 2 is equipped with a temperature adjustment device 21 for increasing and reducing the temperature inside the culture chamber; a humidifier 22 for adjusting the humidity; a gas feed device 23 for supplying CO₂ gas, N₂ gas, or other gases; a circulation fan 24 for keeping the overall environment of the culture chamber 2 uniform; an environment sensor 25 for detecting the temperature, humidity, and the like of the culture chamber 2; and other components. The operation of the devices is controlled by the control unit 6; and the culture environment specified by the temperature, humidity, carbon dioxide concentration, and the like of the culture chamber 2 is kept in a state that is consistent with the culture conditions configured using the control board 7.

The stocker 3 is formed with a plurality of shelves partitioned vertically and perpendicularly with respect to the plane of the diagram of FIG. 2. Each of the shelves is given a unique number. For example, assuming that columns A though C are arranged in the perpendicular direction, and shelves 1 through 7 are arranged in the vertical direction, then the shelf in column A, row 5 is designated as "A-5."

The culture containers 10 may be a flask, dish, well plate, or of another type; may be round, angular, or otherwise configured; and are of appropriate size. They may be suitably selected and used in accordance with the purpose and species of the cells to be cultured. A configuration in which a dish is used has been given byway of example in the present embodiment. Samples of cells or the like are injected into the culture containers 10 together with a liquid culture medium containing phenol red or another pH indicator. Code numbers are assigned to the culture containers 10 and are stored in accordance with the assigned address in the stocker 3. Container holders for use in transportation, formed in accordance with the type, form, or other parameters of the containers, are mounted on the culture containers 10 and held on the shelves.

A transport unit 4 is composed of a Z stage 41 that is raised by a Z-axis drive mechanism and provided so as to allow movement in the vertical direction inside the culture chamber 2; a Y stage 42 that is moved in the perpendicular direction by a Y-axis drive mechanism and mounted on the Z stage 41 so as to allow movement in the perpendicular direction; an X stage 43 that is moved in the lateral direction by an X-axis drive mechanism and mounted on the Y stage 42 so as to allow movement in the lateral direction; and other components. A support arm 45 for lifting and supporting the culture containers 10 is provided to the distal end side of the X stage 43, which is moved in the lateral direction in relation to the Y stage. The transport unit 4 is configured so that the support arm 45 has a movement range that allows movement between all the shelves of the stocker 3 and a sample stage 15 in the observation unit 5. The X-axis drive mechanism, the Y-axis drive mechanism, and the Z-axis drive mechanism are composed of, e.g., servo motors with a ball screw and encoder, and the operation of the drive mechanisms is controlled by the control unit 6.

The observation unit 5 is composed of a first illumination section 51, a second illumination section 52, a third illumination section 53, a macro viewing system 54 for observing samples macroscopically, a microscope viewing system 55 for observing samples microscopically, an image processing device 100, and other components. The sample stage 15 is composed of a translucent material, and a transparent window section 16 is provided to the observation region of the microscope viewing system 55.

The first illumination section 51 is composed of a plane-emission light source provided to a lower frame 1 b, and provides backlighting to all the culture containers 10 from the lower side of the sample stage 15. The second illumination section 52 has an illumination system composed of LEDs or another light source, and a phase ring, condenser lens, and the like; is provided to the culture chamber 2; and is used for illuminating the samples in the culture containers from above the sample stage 15 along the optical axis of the microscope viewing system 5. The third illumination section 53 has an illumination optical system composed of a plurality of LEDS, a mercury lamp, or other light sources for emitting light at a wavelength suited to epi-illumination observation or fluorescent observation; and a beam splitter, a fluorescent filter, or the like for superimposing light emitted from the light sources onto the optical axis of the microscope viewing system 55. The third illumination section 53 is disposed inside the lower frame 1 b positioned on the lower side of the culture chamber 2; and illuminates the samples in the culture containers along the optical axis of the micro observation unit 5 from below the sample stage 15.

The macro viewing system 54 has an observation optical system 54a and a CCD camera or another imaging device 54c for photographing representations of the samples imaged by the observation optical system, and is disposed inside the culture chamber 2 positioned above the first illumination section 51. The macro viewing system 54 photographs an overall observation image (macro representation) from above the culture containers 10 backlit by the first illumination section 51.

The microscope viewing system 55 is disposed inside the lower frame 1b, and has an observation optical system 55a composed of an objective, a middle zooming lens, a fluorescent filter, and the like; and a cooled CCD camera or another imaging device 55c for photographing representations of the samples imaged by the observation optical system 55a. The objective and middle zooming lens are provided in a plural number, and are configured so that a plurality of magnifications can be set using a revolver, a slider, or another displacement mechanism (not shown) and the magnification can be varied within a range of, e.g., 2x to 80× in accordance with an initially selected lens setting. The microscope viewing system 55 captures a microscopically observed representation (i.e., a micro representation), obtained by microscopically observing transmitted light illuminated by the second illuminating part 52 and transmitted through the cell, reflected light illuminated by the third illuminating part 53 and reflected by the cell, or fluorescent light emitted by the cell when illumination has been provided by the third illuminating part 53.

The image-processing device 100 performs an analog-to-digital conversion on a signal inputted from the imaging device 54c of the macro viewing system and the imaging device 55c of the microscope viewing system, performs a variety of types of image processing, and generates image data for the overall observed image or the microscopically observed image. The image-processing device 100 also performs image analysis on the image data for the observed images, generates a time-lapse image, calculates cell travel, analyzes the cell movement state, or performs other tasks. Specifically, the image-processing device 100 is configured by executing an image-processing program stored in a ROM of the control unit 6 described below. The image-processing device 100 will be described in detail further below.

The control unit 6 comprises a CPU 61; a ROM 62 having configured and stored therein a control program for controlling the operation of the culture observation system BS, or data for controlling a variety of components; a RAM 63 for temporarily storing image data and other data; and other devices. In the control unit 6, the devices are connected by a data bus. Connected to an input/output port of the control unit 6 are the temperature regulating device 21, the humidifier 22, the gas feed device 23, the circulation fan 24, and the environment sensor 25 provided to the culture chamber 2; each of the X-, Y-, and Z-axis driving mechanisms for driving the X, Y, Z stages 43, 42, 41 provided to the transport unit 4; the first, second, and third illumination sections 51, 52, 53, the macro viewing system 54, and the microscope viewing system 55 provided to the observation unit 5; a control panel 71 and a display panel 72 provided to the control board 7; and other devices. A detection signal is inputted from each of the devices listed above into the CPU 61, and each of the devices is controlled in accordance with a control program stored in advance in the ROM 62.

The control panel 7, to which is provided a keyboard, a sheet switch, and an input/output device such as a read/write device for reading information from, and writing information to, a magnetic recording medium, an optical disc, or another medium; and the display panel 72, for displaying a variety of operation screens, image data, and other information, are provided to the control board 7. The user configures an observation program (operating conditions), selects conditions, and enters an operation command or other information using the control panel 71 while referring to the display panel 72, and thereby operates, via the CPU 61, the devices provided to the culture observation system BS. In other words, in accordance with what is inputted from the control panel 71, the CPU 61 adjusts the environment in the culture chamber 2; transports the culture container 10 within the culture chamber 2; observes the sample using the observation unit 5; analyzes obtained image data; displays the image data on the display panel 72; and performs other operations. The display panel 72 displays numerical values representing environmental conditions in the culture chamber 2, analyzed image data, alerts in the event of a fault, and the like in addition to other input screens for operation commands, condition selections, and the like. The CPU 61 is able to transmit and receive data to and from an externally connected computer or another device via a communication section 65 compliant with wired or wireless telecommunication standards.

The temperature, humidity, or other environmental conditions in the culture chamber 2; an observation schedule for each of the culture containers 10; the type, position, magnification, and other observation conditions associated with the observation unit 5; and other operation conditions for the observation program configured using the control panel 71 are stored in the RAM 63. The code number for each of the culture containers 10 accommodated in the culture chamber 2, the storage address of the culture container 10 in the stocker 3 corresponding to each code number, and other management data for managing the culture container 10; and a variety of data used for the image analysis are also stored in the RAM 63. The RAM 63 is provided with an image data storage region for storing data relating to images captured by the observation unit 5. Indexing data, containing the code number of the culture container 10, the date and time when the image was captured, and similar information, is stored in correlation with the image data.

In the culture observation system BS configured as above, the CPU 61 controls the operation of each of the devices based on the control program stored in the ROM 62 and automatically captures an image of the sample in the culture container 10, according to the conditions set for the observation program as entered using the control board 7. In other words, when operation of the control panel 71 (or remote operation via the communication section 65) starts the observation program, the CPU 61 reads the value of each of the environmental conditions stored in the RAM 63; detects the environmental state in the culture chamber 2 inputted from the environment sensor 25; operates the temperature adjustment device 21, the humidifier 22, the gas feed device 23, the circulation fan 24, and similar devices according to the difference between the condition value and the actual value; and performs feedback control on the temperature, humidity, carbon dioxide concentration, and other culture environment conditions in the culture chamber 2.

The CPU 61 reads the observation conditions stored in the RAM 63, operates each of the X-, Y-, and Z-axis driving mechanisms for driving the X, Y, Z stages 43, 42, 41 provided to the transport unit 4 and transports the culture container 10 corresponding to the observed object from the stocker 3 to the sample stage 15 in the observation unit 5 according to an observation schedule, and starts observation of the observed object by the observation unit 5. For example, in an instance where the observation program has been set for macroscopic viewing, the culture container 10 conveyed by the conveying unit 4 from the stocker 3 is positioned on an optical axis of the macro viewing system 54 and placed on the sample stage 15, the light source of the first illuminating part 51 is illuminated, and the imaging device 54c is used to capture an overall observed representation from above the backlit culture container 10. A signal sent from the imaging device 54c into the control unit 6 is processed by the image-processing device 100, an overall observed representation is generated, and the image data is stored in the RAM 63 together with the indexing data, such as the date and time when the image was captured, and other information.

In an instance where the observation program has been set for microscopic viewing of a sample at a specific location in the culture container 10, the specific location in the culture container 10 transported by the transport unit 4 is positioned on an optical axis of the microscope viewing system 55 and placed on the sample stage 15, the light source of the second illuminating part 52 or the third illuminating part 53 is illuminated, and the imaging device 55c is used to capture a transmission-illuminated, epi-illuminated, or fluorescence-assisted microscopically observed representation. A signal obtained when an image is captured by the imaging device 55c and sent to the control unit 6 is processed by the image-processing device 100, a microscopically observed representation is generated, and the image data is stored in the RAM 63 together with the indexing data, such as the date and time when the image was captured, and other information.

The CPU 61 performs the observation described above on a plurality of samples in culture containers accommodated in the stocker 3, wherein the overall observed representation or the microscopically observed representation is successively captured according to an observation schedule having a time interval of about 30 minutes to 2 hours based on the observation program. According to the present embodiment, the time interval between captured images may be fixed or variable. The image data for the overall observed representation or the microscopically observed representation that has been captured is stored together with the code number of the culture container 10 in the image data storage region of the RAM 63. The image data stored in the RAM 63 is read from the RAM 63 according to an image display command inputted from the control panel 71, and an overall observed representation or a microscopically observed representation for a specified time (i.e., a single image), or a time-lapse image of overall observed representations or microscopically observed representations from a specified time region, are displayed on the display panel 72 of the control board 7.

### (Method for predicting cell movement)

In the culture observation system BS thus configured, in addition to generation of time lapse images, cell tracking, and other functions, the image processing device 100 is provided with a function for predicting the movement direction of cells. As methods for predicting the movement of cells, the movement prediction methods presented in this specification include a method for predicting movement by extracting a shape characteristic of a cell to be observed (observed cell) from an image containing the observed cell, and a method for predicting movement by extracting the characteristics of the internal structure of an observed cell. Described hereinbelow are the basic concepts of I) movement prediction based on a shape characteristic, and II) movement prediction based on the characteristics of the internal structure.

### (Preprocessing)

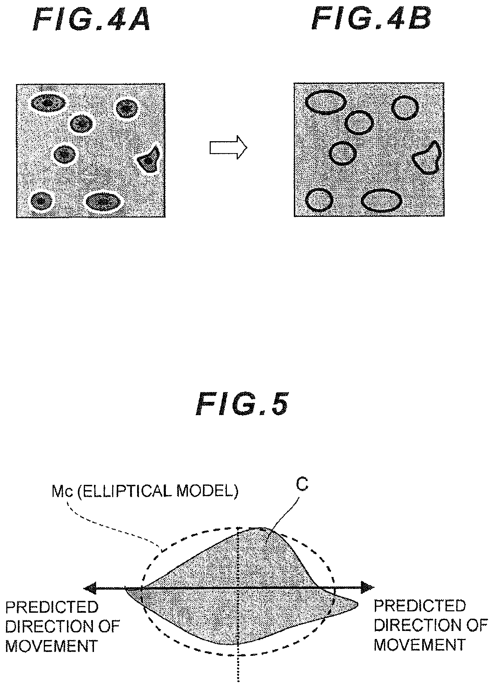

Prior to movement prediction processing, the outermost contour of cells is extracted. FIG. 4 is a schematic view exemplifying the state of the process for extracting the outermost contour, wherein image (a) obtained by the imaging device 55c (54c) is processed, and the outermost contour of the cells is extracted as shown in (b). Examples of the contour extraction process include a method for forming binary data from luminance brightness values, a method for forming binary data from dispersion values, and a dynamic contour extraction method such as Snakes, Level Set, and the like. In the present specification, the term "observed cell" is used to describe a cell which is to be observed and for which movement prediction is to be carried out.

### (I: Movement prediction based on a shape characteristic)

To predict the movement of a cell on the basis of a shape characteristic of the cell, a cell model made by modeling the outer shape of the cell is adapted to the observed cell from which the outermost contour has been extracted by the preprocessing, and a movement direction in which the observed cell is predicted to move is derived using the adapted cell model. Examples of specific prediction methods that include such movement predictions include methods in which the movement direction is predicted (1) based on a shape characteristic of a cell model adapted to the observed cell, (2) based on the offset between the centrobaric position of the cell model adapted to the observed cell and the centrobaric position of the observed cell extracted from the contour, and (3) based on the offset between the contour shape of the cell model adapted to the observed cell and the contour shape of the observed cell.

To adapt the cell model to an observed cell, the outermost contour of the observed cell C extracted by the preprocessing is approximated to an elliptical model, as shown by the example of adapting the elliptically shaped cell model Mc in FIGS. 5, 6, and 1, for example. The least squares method and methods based on moment calculations can be cited as examples of the elliptical approximation referred to in this case. A model in which the contour interior of the observed cell C is embedded may also be estimated to be an elliptical model of the highest correlation. The predicted direction of cell movement can be estimated using this cell model Mc.

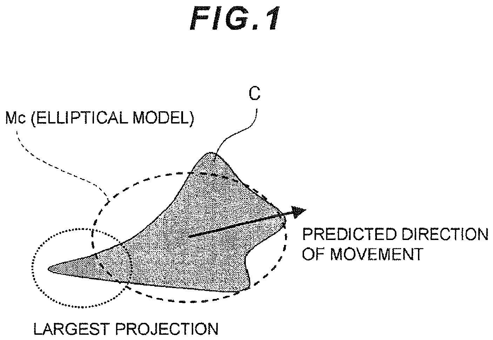

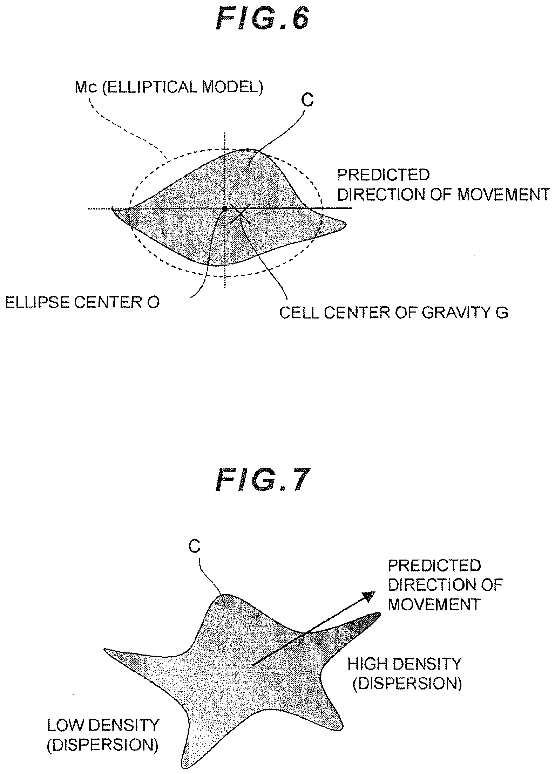

In the prediction method based on a shape characteristic of cell model (1), the longitudinal axial direction (the arrowed direction in FIG. 5) of the ellipse of the cell model Mc is derived as the predicted movement direction of the observed cell C. Specifically, it is usually highly probable that the movement direction of the cell is the longitudinal direction, i.e., the longitudinal axial direction of the approximated ellipse, in the case of a cell such as one approximated to an elliptical shape whose aspect ratio is at or above a certain level. In this prediction method, the movement direction is assessed using the relationship between a shape characteristic and the movement direction of such a cell. It is impossible in this case to directly derive whether the observed cell C will move to the left or right along the longitudinal axis of the ellipse, but this approach can be considered to be able to markedly reduce the angular range during calculation of cell tracking and to provide sufficient information for a preliminary prediction.
(2) In the prediction method based on the offset between the centrobaric position of the cell model and the centrobaric position of the observed cell, a cell model Mc is applied to the observed cell C, the centrobaric position G of the observed cell C is determined by the graphical processing of the contour shape thereof, and the movement direction of the observed cell is derived from the offset between the ellipse center O of the cell model Mc and the centrobaric position G of the observed cell C, as shown in FIG. 6. In this method, the movement direction is assessed using a characteristic according to which the centrobaric position of a cell is biased in the movement direction (or in the opposite direction) in the case of a cell that moves while changing its shape. For example, the bias direction of the center of gravity G of the observed cell can be derived as a predicted movement direction in cases in which the center of gravity G is biased to the right from the center O of the elliptically approximated cell model Mc, as shown in FIG. 6.
(3) In the prediction method based on the offset between the contour shape of the cell model and the contour shape of the observed cell, a case in which the cell contour is complicated in comparison with the elliptical shape of the cell model Mc can be suggested as a more complicated case, as shown in FIG. 1. In this case, the orientation that has areas with the largest projection distance is predicted to be the direction of movement of the observed cell C, or the orientation on the opposite side of the interposed ellipse center O is predicted to be the direction of movement of the observed cell C, based on the position and size of the cell contour that projects from the cell model Mc. Either of the cases is obtained using the characteristic that a part (bottom) of the body is extended during cell movement, or the characteristic that an adhesive surface is left behind during movement for some cells. Which of the two directions is the movement direction of the cell is determined by the type of cell.

An elliptical shape was shown as an example of the structure of the cell model Mc, but any other suitable shape can also be used in accordance with the morphological characteristics of the cell being observed. Examples of such shapes include triangular shapes, rectangular shapes, star shapes, arcuate shapes, and the like.

### (II: Movement prediction based on internal structure characteristics)

To predict the movement of a cell on the basis of the internal structure characteristics of the cell, bias is computed for the internal structure in relation to the contour shape of the observed cell from which the outermost contour has been extracted, and a movement direction that the observed cell is predicted to move is derived on the basis of the computed bias of the internal structure. A method based on the cell density of the observed cell, and a method based on a texture characteristic of the observed cell are proposed as specific methods for detecting the bias of an internal structure.

The method based on the density of the internal structure will first be cited as a method for determining the bias in the internal structure of an observed cell. Cell density can be expressed using the dispersion of luminance brightness values inside the cell contour in a microscopically viewed image. In view of this, cell density in a cell contour is determined based on the dispersion of the luminance brightness values, the orientation from the areas of low cell density to the areas of high cell density is computed, and the direction of this orientation, or a direction reverse to this orientation, is adopted as the predicted direction of movement, as shown in FIG. 7. In this movement prediction method, the predicted direction of cell movement is assessed using the fact that when a cell moves, there is a tendency for the movement to occur in the direction in which the internal tissue is caused to move, or in the reverse direction. The relation between the direction of change in density and the direction of cell movement are determined by the type of cell.

The method based on a texture characteristic of the observed cell can also be cited as a method for determining the bias in the internal structure of an observed cell. A nucleus, internal tissue, and other types of texture can be found in a cell, and these internal structures move or change during cell movement. In view of this, the texture inside the cell contour is determined in this movement prediction method by using a dispersion filter or the like. For example, the movement direction can be predicted based on the position of the nucleus within the cell contour, the direction of extension of the internal tissue, or the like.

### (Examples)

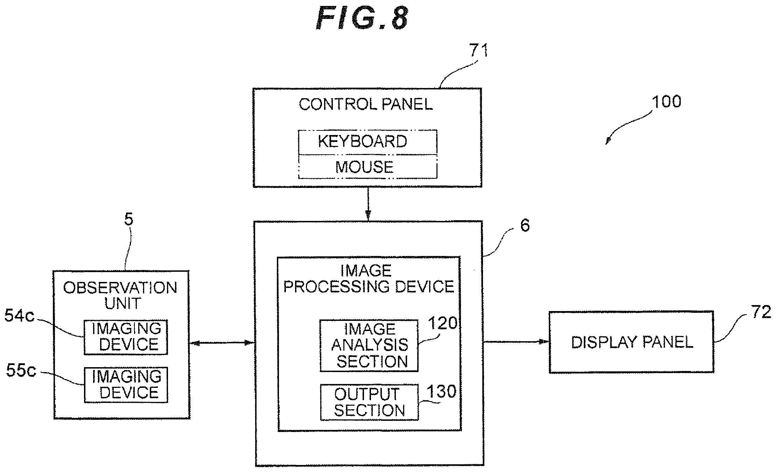

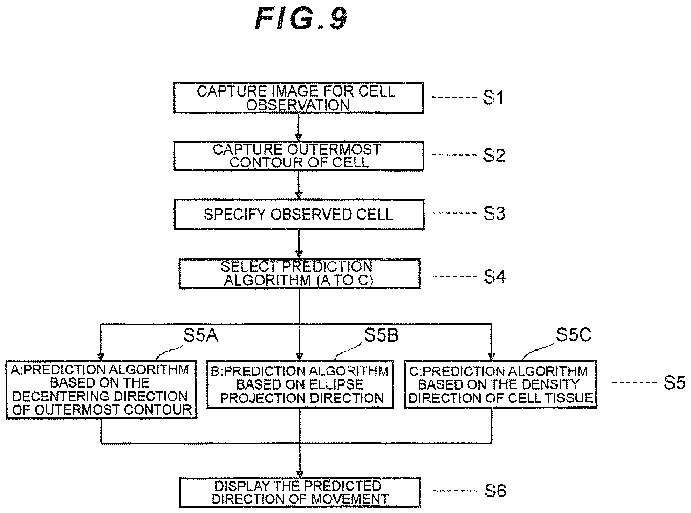

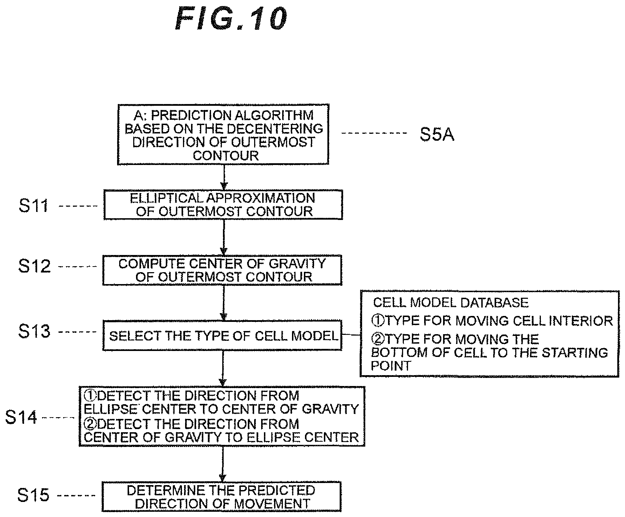

Next, a specific application of image analysis carried out by the image processing device 100 of the culture observation system BS will be described with reference to FIGS. 8 through 12. Here, FIG. 8 is a block view showing the general configuration of the image processing device 100 for carrying out image processing for movement prediction, FIG. 9 is a flowchart showing the main flow in the image processing program GP for movement prediction, and FIGS. 10 through 12 are flowcharts that correspond to prediction algorithms A, B, and C selected in the main flow.

The image processing device 100 is provided with an image analysis section 120 for obtaining an observation image obtained by photographing an observed cell C by the imaging device 55c (54c), and deriving a movement direction in which the observed cell is predicted to move, and an output section 130 for outputting to the exterior the movement direction of the observed cell C as derived by the image analysis section 120; and is configured so as that the predicted movement direction derived by the image analysis section 120 is output and displayed to, e.g., the display panel 72. The image processing device 100 is configured so that the image processing program GP stored in advance in the ROM 62 is read by the CPU 61, and processing based on the image processing program GP is sequentially carried out by the CPU 61.

In view of the above, when algorithm A or B is selected in the main flow of the image processing program GP, the image analysis section 120 extracts the contour of the observed cell C from the obtained observation image, adapts the cell model Mc made by modeling the outer shape of the cell to the observed cell C from which the contour has been extracted, and derives the predicted movement direction of the observed cell C using the adapted cell model Mc (see FIGS. 4 to 6, and FIG. 1). On the other hand, when algorithm C is selected in the main flow of the image processing program GP, the image analysis section 120 extracts the contour of the observed cell C from the obtained observation image, computes the bias of the internal structure in relation to the contour shape of the observed cell C from which the contour has been extracted, and derives the movement direction in which the observed cell C is predicted to move on the basis of the computed bias of the internal structure (see FIGS. 4 and 7).

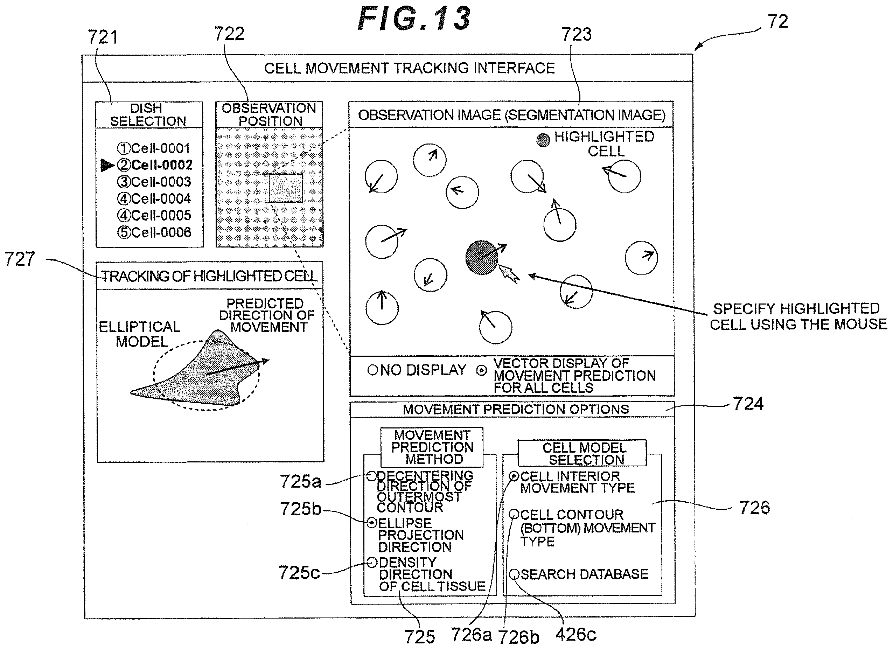

The image analysis processing performed by the image analysis section 120 as described above may also be carried out by reading the image data of an observation image containing the observed cell C already stored in the RAM 63, or may be carried out by using an imaging device to capture an image of a cell for which observation is to be initiated. In view of this, a case in which movement is predicted by obtaining a current image will be described in the present embodiment with reference to FIG. 13, which shows a configuration example of the display image of the movement prediction interface on the display panel 72.

In this interface, when "cell movement prediction" is selected and executed via the control panel 71, first, a "dish selection" frame 721 is displayed on the display panel 72, a list of code numbers of the culture containers 10 stored in the stocker 3 is displayed, and the culture container 10 to be observed is selected. FIG. 13 shows the state in which a culture cell dish (culture container) having the code number Cell-0002 is selected by the cursor provided to the control panel 71.

When the culture container has been selected, the CPU 61 actuates the drive mechanisms of the shafts of the transport unit 4 to transport the culture container 10 to be observed from the stocker 3 to the observation unit 5, causes the imaging device 55c to photograph the microscopic viewing image produced by the microscopic viewing system 55, and displays the resulting image in an "observation position" frame 722.

Next, the region of the observation image is set in order to capture the observation image containing the cell to be observed (observed cell). FIG. 13 shows a state in which an observer has specified a shaded region in the center right area using a mouse provided to the control panel 71. An image of the region specified by the observer is thereby obtained by the image analysis section 120 as an observation image (step S1).

A process (step S2) for extracting (segmenting) the outermost contour of a cell from the obtained observation image is instantaneously carried out by the image analysis section 120, and the image of the cell from which the outermost contour has been extracted is displayed in an "observation image" frame 723 of the display panel 72. The observed cell (highlighted cell) for which the movement direction is to be predicted is then specified (step S3) in the observation image using a mouse or the like, as shown in FIG. 13. The observed cell may be specified to be a cell already detected by tracking or the like. In this case, a "movement prediction option" frame 724 is formed below the observation image frame 723, a "movement prediction method" frame 725 is formed inside the frame 724, and the movement prediction method selection buttons 725a, 725b, 725c are displayed for selecting which prediction algorithm is to be used for predicting movement.

The prediction algorithms in the illustrated examples are configured so that a prediction method can be selected from the following three types of methods:

### A: Prediction using the decentering direction of the outermost contour

Specifically, this is a method for predicting movement direction on the basis of the offset between the centrobaric position O of the cell model Mc and the centrobaric position G of the observed cell C described with reference to FIG. 6 in section (2) of I: Movement prediction based on a shape characteristic

### B: Prediction using the ellipse projection direction

Specifically, this is a method for predicting the movement direction on the basis of the offset between the contour shape of the cell model Mc and the contour shape of the observed cell C as described with reference to FIG. 1 in section (3) of I: Movement prediction based on a shape characteristic

### C: Prediction using the density direction of cell tissue

Specifically, this is a method for predicting the movement direction on the basis of the characteristics of the internal structure of the observed cell C as described with reference to FIG. 7 in II: Movement prediction based on the internal structure characteristics.

In this configuration, the following buttons are displayed on the main screen: (A) selection button 725a for the prediction method based on the decentering direction of the outermost contour, (B) selection button 725b for the prediction method using the ellipse projection direction, and (C) selection button 725c for the prediction method using the density direction of cell tissue

In step S4, the observer selects any of the selection buttons 725a, 725b, 725c to select a prediction algorithm A, B, or C. In accordance with this selection, processing flow is branched in step S5 to flow S5A of the prediction algorithm based on the decentering direction of the outermost contour, to flow S5B of the prediction algorithm based on the ellipse projection direction, or to flow S5C of the prediction algorithm based on the density direction of cell tissue.

### (S5A: Flow of prediction algorithm based on the decentering direction of outermost contour)

In the prediction algorithm based on the decentering direction of the outermost contour, first, as shown in FIG. 10, a process for approximating the cell model Mc with respect to the outermost contour of the observed cell is carried out in step S11, and the elliptically shaped cell model Mc is adapted to the observed cell C, as shown by the dotted line in FIG. 6, for example. Next, the centrobaric position (the center position in the case of an ellipse) O of the cell model Mc and the centrobaric position G in the contour shape of the observed cell C are computed in step S12, and the process then proceeds to step S13.

In step S13, a "cell model selection" frame 726 is formed in the "movement prediction option" frame 724, and selection buttons for selecting the movement characteristics of the observed cell C are displayed inside the frame. In this case, the "cell internal movement" selection button 726a for moving the centrobaric position during movement, or the "cell contour (bottom) movement" selection button 726b for extending a portion of the body is selected. In correspondence to these selection buttons, submenus are displayed (not shown) for selecting a type for biasing the center of gravity in the movement direction of the cell, or a reverse type (726a), a type for extending the bottom or the type in which an adhesive surface is left behind (726b), or other types; and the movement characteristics of the observed cell C are specified.

In relation to the movement characteristics of a cell, a database is constructed for associating and registering in advance a cell name (species, identification number, and the like) and the movement characteristics, a database search button 726c is selected, and the name of the observed cell is inputted, whereby an item is provided for automatically searching the database and determining the movement characteristics of the cell. The inputting burden on the observer can thereby be reduced, selection errors can be prevented, and the accuracy of movement prediction can be improved.

Next, in step S14, the movement direction is detected in accordance with the movement characteristics of the cell selected in step S13. For example, the positional relationship between the center of gravity G of the observed cell C and the center O of the cell model Mc computed in step S12 is computed when the center of gravity G of the observed cell C is positioned to the right of the center O of the cell model Mc, as shown in FIG. 6, and the direction facing from the ellipse center O of the cell model toward the center of gravity G of the observed cell is computed in step S14 in the case that the movement characteristics of the cell selected in step S13 are characteristics for biasing the center of gravity G in the movement direction. On the other hand, the direction facing from the center of gravity G of the observed cell toward the ellipse center O of the cell model is computed in step S14 in the case that the movement characteristics of the cell selected in step S13 are characteristics for biasing the center of gravity G in the direction opposite to the movement direction even when the positional relationship between the center of gravity G of the observed cell C and the center O of the cell model Mc computed in step S12 is the same. The direction computed in step S14 is determined to be the predicted movement direction of the observed cell C in step S15, and the process returns to the main flow for movement prediction and then advances to step S6.

In step S6, a "highlighted cell tracking" frame 727 is formed on the display screen, and a close-up display of the observed cell C specified in step S3 and the predicted movement direction of the observed cell C are displayed in this frame by a vector display. In the case that movement prediction is to be carried out for all cells positioned inside the observation image, the selection button for "vector display of movement prediction for all cells" formed below the "observation image" frame 723 is switched on, whereby the movement vector is superimposed and displayed for each of the cells in the observation image, as shown in the drawing.

### (S5B: Flow of prediction algorithm based on ellipse projection direction)

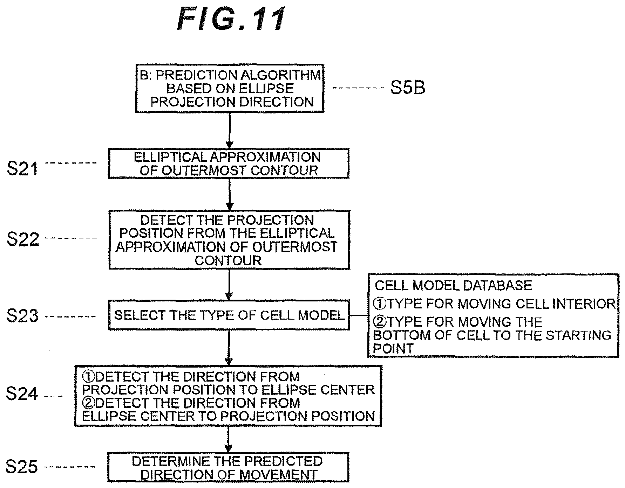

In the prediction algorithm based on the ellipse projection direction, a process for approximating the cell model Mc with respect to the outermost contour of an observed cell is carried out in step S21, as shown in FIG. 11, and the elliptically shaped cell model Mc is adapted to the observed cell C, as shown by the dotted line in FIG. 1, for example. Next, the size and position of the observed cell C that projects from the cell model Mc are computed in step S22, the direction in which the projection distance is maximum is computed, and the process proceeds to step S23.

In step S23, the "cell model selection" frame 726 is formed in the "movement prediction option" frame 724 of the display screen, and selection buttons for selecting the movement characteristics of the observed cell C are displayed. The observer selects either the "cell internal movement" selection button 726a or the "cell contour (bottom) movement" selection button 726b in accordance with the observed cell in the same manner as described above. The type for biasing the center of gravity in the movement direction of the cell or a reverse type (726a), the type for extending the bottom or the type in which an adhesive surface is left behind (726b), or the like are selected from the submenus (not shown) displayed in accordance with the selected button, whereby the movement characteristics of the observed cell C are selected. In the same manner as described above, a database is constructed for the movement characteristics of a cell, a database search button 726c is selected, and the name of the observed cell is inputted, whereby an item is also provided for automatically determining the movement characteristics of the cell. The inputting burden on the observer can thereby be reduced, selection errors can be prevented, and the accuracy of movement prediction can be improved.

In step S24, the movement direction is detected in accordance with the movement characteristics of the cell selected in step S23. For example, the largest projection of the observed cell C in relation to the cell model Mc computed in step S22 is computed to be to the lower left of the cell model Mc, as shown in FIG. 1, and the direction facing from the projection position toward the ellipse center is computed in step S24 in the case that the movement characteristics of the cell selected in step S23 are characteristics according to which an adhesive surface is left behind during movement. On the other hand, when the largest projection of the observed cell C in relation to the cell model Mc computed in step S22 is the same, the direction facing from the ellipse center toward the projection position is computed in step S24 in the case that the movement characteristics of the cell selected in step S23 are characteristics according to which the bottom is extended in the movement direction during movement. The direction computed in step S24 is determined to be the predicted movement direction of the observed cell C in step S25, and the process returns to the main flow for movement prediction and then advances to step S6.

In step S6, the "highlighted cell tracking" frame 727 is formed on the display screen, and a close-up display of the observed cell C specified in step S3 and the predicted movement direction of the observed cell are displayed in this frame by a vector display such as in the drawing. In the case that movement prediction is to be carried out for all cells positioned inside the observation image, the selection button for "vector display of movement prediction for all cells" formed below the "observation image" frame 723 is switched on, whereby the movement vector is superimposed and displayed for each of the cells in the observation image, as shown in the drawing.

### (S5C: Flow of prediction algorithm based on the density direction of cell tissue)

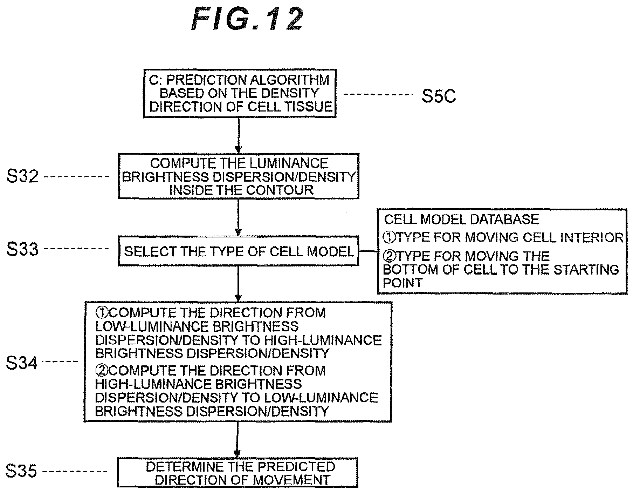

In the prediction algorithm based on the density direction of cell tissue, first, the density distribution inside the cell is computed in step S32 by computing the dispersion of luminance brightness values inside the observed cell contour, as shown in FIG. 12. The distribution of low-density regions and high-density regions of the cell is thereby computed from the structural characteristics of the cell interior even when the cell has a complex contour shape, as shown in FIG. 7. The process then proceeds to step S33.

In step S33, the "cell model selection" frame 726 is formed in the "movement prediction option" frame 724 on the display screen, and selection buttons for selecting the movement characteristics of the observed cell C are displayed. In this case, either the "high-density direction movement" selection button in which the density in the movement direction increases during movement, or the "low-density direction movement" selection button in which the density in the movement direction decreases during movement is selected (these selection buttons are not shown). In the same manner as described above, a database is constructed for the movement characteristics of a cell, a database search button 726c is searched, and the name of the observed cell is inputted, whereby an item is also provided for automatically determining the movement characteristics of the cell. The inputting burden on the observer can thereby be reduced, selection errors can be prevented, and the accuracy of movement prediction can be improved.

In step S34, the movement direction is detected in accordance with the movement characteristics of the cell selected in step S33. For example, the density distribution inside the cell is computed in step S32 and the density is computed to be high in the upper right area and low in the lower left area of the observed cell C, as shown in FIG. 7. In the case that the movement characteristics of the cell selected in step S33 are of a high-density direction movement type in which the cell is moving in the direction of high density, the direction facing from the region of low density toward the region of high density is computed in step S34. On the other hand, in the case that the density distribution is the same but the movement characteristics of the cell selected in the step S33 are of a low-density direction movement type in which the cell is moving in the direction of low density, the direction facing from the region of high density toward the region of low density is computed in step S34. The direction computed in step S34 is determined to be the predicted movement direction of the observed cell C in step S35, and the process returns to the main flow for movement prediction and then advances to step S6.

In step S6, a "highlighted cell tracking" frame 727 is formed on the display screen, and a close-up display of the observed cell C specified in step S3 and the predicted movement direction of the observed cell C are displayed in this frame by a vector display. In the case that movement prediction is to be carried out for all cells positioned inside the observation image, the selection button for "vector display of movement prediction of all cells" formed below the "observation image" frame 723 is switched on, whereby the movement vector is superimposed and displayed for each of the cells in the observation image, as shown in the drawing.

Therefore, an advantageous prediction algorithm can be selected and used in accordance with the characteristics of the observation target. The observer can ascertain in detail the movement direction of a cell being observed by viewing the "highlighted cell tracking" frame 727 when any of the prediction algorithms has been selected, and can ascertain the movement state of the cells of an entire observation region by superimposing and displaying the movement vector for each of the cells in the observation image in the "observation image" frame 723.

As described above, in accordance with the image processing program GP, the method for analyzing an image by executing the image processing program, and the image processing device 100 of the present invention, the movement direction of a cell can be predicted from a single image currently or already recorded, without reading and processing numerous images photographed and recorded by an imaging device. Therefore, it is possible to provide means capable of predicting cell movement and carrying out high-speed prediction processing with a very simple configuration.
The description further comprises the following clauses:
1. A method for analyzing an image for cell observation, comprising:
   obtaining an observation image in which an observed cell has been photographed by an imaging device;
   extracting a contour of the observed cell from the obtained observation image;
   adapting a cell model made by modeling an outer shape of the cell to the observed cell from which the contour has been extracted; and
   deriving a movement direction in which the observed cell is predicted to move, using the adapted cell model.
2. The method for analyzing an image for cell observation according to clause 1, wherein the movement direction is derived based on a shape characteristic of the adapted cell model.
3. The method for analyzing an image for cell observation according to clause 1, wherein the movement direction is derived based on an offset between the centrobaric position of the adapted cell model and the centrobaric position of the observed cell from which the contour has been extracted.
4. The method for analyzing an image for cell observation according to clause 1, wherein the movement direction is derived based on an offset between the contour shape of the adapted cell model and the contour shape of the observed cell.
5. A method for analyzing an image for cell observation, comprising:
   obtaining an observation image in which an observed cell has been photographed by an imaging device;
   extracting a contour of the observed cell from the obtained observation image;
   computing a bias of the internal structure in relation to the contour shape of the observed cell from which the contour has been extracted; and
   deriving a movement direction that the observed cell is predicted to move based on the computed bias of the internal structure.
6. The method for analyzing an image for cell observation according to clause 5, wherein the bias of the internal structure is a cell density of the observed cell.
7. The method for analyzing an image for cell observation according to clause 5, wherein the bias of the internal structure is a texture characteristic of the observed cell.
8. A program for processing an image for cell observation, comprising:
   obtaining an observation image in which an observed cell has been photographed by an imaging device;
   extracting a contour of the observed cell from the obtained observation image;
   adapting a cell model made by modeling an outer shape of the cell to the observed cell from which the contour has been extracted;
   deriving a movement direction in which the observed cell is predicted to move, using the adapted cell model; and
   outputting to the exterior the derived movement direction of the observed cell.
9. The program for processing an image for cell observation according to clause 8, wherein the movement direction is derived based on a shape characteristic of the adapted cell model.
10. The program for processing an image for cell observation according to clause 8, wherein the movement direction is derived based on an offset between the centrobaric position of the adapted cell model and the centrobaric position of the observed cell from which the contour has been extracted.
11. The program for processing an image for cell observation according to clause 8, wherein the movement direction is derived based on an offset between the contour shape of the adapted cell model and the contour shape of the observed cell.
12. A program for processing an image for cell observation, comprising:
   obtaining an observation image in which an observed cell has been photographed by an imaging device;
   extracting a contour of the observed cell from the obtained observation image;
   computing a bias of the internal structure in relation to the contour shape of the observed cell from which the contour has been extracted;
   deriving a movement direction in which the observed cell is predicted to move based on the computed bias of the internal structure; and
   outputting to the exterior the derived movement direction of the observed cell.
13. The program for processing an image for cell observation according to clause 12, wherein the bias of the internal structure is a cell density of the observed cell.
14. The program for processing an image for cell observation according to clause 12, wherein the bias of the internal structure is a texture characteristic of the observed cell.
15. An image processing device for cell observation, comprising:
   an imaging device configured to photograph an observed cell;
   an image analysis section obtaining an observation image in which the observed cell has been photographed by the imaging device, and deriving a movement direction in which the observed cell is predicted to move; and
   an output section outputting to the exterior the movement direction of the observed cell derived by the image analysis section, wherein
   the image analysis section is configured to extract a contour of the observed cell from the observation image, adapt a cell model made by modeling an outer shape of the cell to the observed cell from which the contour has been extracted, and derive a movement direction in which the observed cell is predicted to move, using the adapted cell model.
16. The image processing device for cell observation according to clause 15, wherein the movement direction is derived based on a shape characteristic of the adapted cell model.
17. The image processing device for cell observation according to clause 15, wherein the movement direction is derived based on an offset between the centrobaric position of the adapted cell model and the centrobaric position of the observed cell from which the contour has been extracted.
18. The image processing device for cell observation according to clause 15, wherein the movement direction is derived based on an offset between the contour shape of the adapted cell model and the contour shape of the observed cell.
19. An image processing device for cell observation, comprising:
   an imaging device configured to photograph an observed cell;
   an image analysis section obtaining an observation image in which the observed cell has been photographed by the imaging device, and deriving a movement direction in which the observed cell is predicted to move; and
   an output section outputting to the exterior the movement direction of the observed cell derived by the image analysis section, wherein
   the image analysis section is configured to extract a contour of the observed cell from the observation image, compute a bias of the internal structure in relation to the contour shape of the observed cell from which the contour has been extracted, and derive a movement direction that the observed cell is predicted to move, based on the computed bias of the internal structure.
20. The image processing device for cell observation according to clause 19, wherein the bias of the internal structure is a cell density of the observed cell.
21. The image processing device for cell observation according to clause 19, wherein the bias of the internal structure is a texture characteristic of the observed cell.

## Claims

1. A method for analyzing an image for cell observation, comprising:
obtaining an observation image in which an observed cell has been photographed by an imaging device;
extracting a contour of the observed cell from the obtained observation image;
computing a bias of the internal structure in relation to the contour shape of the observed cell from which the contour has been extracted; and
deriving a movement direction that the observed cell is predicted to move based on the computed bias of the internal structure.

2. The method for analyzing an image for cell observation according to claim 1, wherein the bias of the internal structure is
- a cell density of the observed cell or
- a texture characteristic of the observed cell.

3. A program for processing an image for cell observation, comprising:
obtaining an observation image in which an observed cell has been photographed by an imaging device;
extracting a contour of the observed cell from the obtained observation image;
computing a bias of the internal structure in relation to the contour shape of the observed cell from which the contour has been extracted;
deriving a movement direction in which the observed cell is predicted to move based on the computed bias of the internal structure; and
outputting to the exterior the derived movement direction of the observed cell.

4. The program for processing an image for cell observation according to claim 3, wherein the bias of the internal structure is
- a cell density of the observed cell or
- a texture characteristic of the observed cell.

5. An image processing device for cell observation, comprising:
an imaging device configured to photograph an observed cell;
an image analysis section obtaining an observation image in which the observed cell has been photographed by the imaging device, and deriving a movement direction in which the observed cell is predicted to move; and
an output section outputting to the exterior the movement direction of the observed cell derived by the image analysis section, wherein
the image analysis section is configured to extract a contour of the observed cell from the observation image, compute a bias of the internal structure in relation to the contour shape of the observed cell from which the contour has been extracted, and derive a movement direction that the observed cell is predicted to move, based on the computed bias of the internal structure.

6. The image processing device for cell observation according to claim 5, wherein the bias of the internal structure is
- a cell density of the observed cell or
- a texture characteristic of the observed cell.
